(19) Europäisches Patentamt

European Patent Office

Office européen des brevets

(11) **EP 2 808 009 A1**

(12) **EUROPEAN PATENT APPLICATION**

(43) Date of publication:
**03.12.2014 Bulletin 2014/49**

(51) Int Cl.:
*A61K 8/98* *(2006.01)*        *A61Q 11/00* *(2006.01)*

(21) Application number: **13002760.0**

(22) Date of filing: **28.05.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Vitalgaia SAS
31100 Toulouse (FR)**

(72) Inventor: **The designation of the inventor has not
yet been filed**

(74) Representative: **Gilholm, Stephen Philip
IPheions Intellectual Property
Buzzard Office
The Hawk Creative Business Park
Easingwold,
York YO61 3FE (GB)**

(54) **Toothpaste comprising propolis extract**

(57)        There is described a toothpaste composition comprising from 0.5% w/w to 2% w/w of soft extract propolis wherein the soft propolis extract comprises from 85 to 95% w/w of active material.,

## Description

### Field of the Invention

The present invention relates to a novel toothpaste formulation.

More particularly, the invention relates to a toothpaste formulation comprising a soft extract of propolis.

### Background of the Invention

Propolis is a resinous mixture collected by honey bees from tree buds, sap flows, broken bark or other botanical sources. Vegetable resins are collected by bees and carried to the hive by bees where the resins are mixed with wax to become into propolis.

It is used by bees as a sealant for unwanted open gaps or spaces in a bee hive. Generally, larger spaces are usually filled with beeswax, whereas smaller gaps or spaces (approximately 6mm or less), are filled with propolis.

The colour of propolis may vary depending upon, *inter alia,* its botanical source. Its colour can vary from yellow to black, including tones of brown and green.

Propolis is sticky at and above room temperature, 20°C (68 F). At lower temperatures, it can become hard and very brittle.

Raw propolis of organic quality is usually collected by beekeepers through a method of scraping.

Thus, propolis is a natural and biologic product and is known to have a number of medical applications, including for example, as an antimicrobial, emollient, immunomodulator, antiplaque dental agent, antitumor growth agent and a radioprotector. Although all types of propolis have similar properties, its effect may depend upon, *inter alia,* the source and type, among other factors.

### Summary of the Invention

The present invention provides a toothpaste composition that possesses antimicrobial, antifungal and/or anti-inflammatory properties. These properties make it useful for oral cleaning, prevention of dental caries, periodontopathies and candidiasis.

Thus, according to the invention there is provided a toothpaste composition comprising from 0.5% w/w to 2% w/w of soft extract of propolis wherein the soft propolis extract comprises from 85 to 95% w/w of active material.

Preferably, the toothpaste composition comprises from about 0.5% w/w to about 1.5% w/w of soft extract of propolis, i.e. from about 0.5g to about 1.5g of soft extract of propolis per 100g of toothpaste, or from about 0.7% w/w to about 1.5% w/w of soft extract of propolis, i.e. from about 0.7g to about 1.5g of soft extract of propolis per 100g of toothpaste, or from about 0.8% w/w to about 1% w/w of soft extract of propolis, i.e. from about 0.8g to about 1g of soft extract of propolis per 100g of toothpaste.

The content of active material (C%) of the soft propolis extract may be determined as follows:

$$C\% = (RP - R)/PSE$$

wherein RP is the weight of raw propolis;

R is the weight of residue after extraction of the raw propolis [obtained after alcohol evaporation]; and

PSE is the weight of the soft extract.

As hereinbefore described the value of C% should be from about 85% to about 90% w/w.

Advantageously, the toothpaste of the invention generally does not require other conventionally used additives, such as, extracts of natural fruit juices, traditional Chinese components, weed extracts, etc. More specifically, the toothpaste of the invention does not contain other additives such as extract of natural fruit juice, traditional Chinese components, weed extracts with coenzyme Q10, extract of pommel seeds, particles of silver and gold, cane and bamboo salt dust, dust of ganoderma, natural sericite, zeolite", dust of green algae, snakes or pearls, carrageen, aloe, farnesol and xylitol, xanthan gum, lemon (essential) oil, tocopherol, pyranose, perfume and/or fluoride.

Depending upon, *inter alia,* the plants and trees in the areas surrounding a beehive, the appearance and composition of raw propolis may vary. Raw propolis is a complex combination of as many as 180 different compounds created from the materials collected by honeybees. The raw materials are further transformed by the bee's saliva as they are harvested and mixed with beeswax when taken back to the beehive.

The toothpaste composition of the invention may include one or more humectants. It will be understood by the person skilled in the art that a humectant is a generally hygroscopic substance used to help a product retain water. When a humectant is present in the toothpaste composition of the invention it will generally be in an amount of from about 10 to about 30% w/w, or about 15 to about 25% w/w, for example about 20% w/w. A variety of humectants may be used in the toothpaste composition and it will be understand that more than one humectant may be used. The humectant used in accordance with the invention is typically a polyol, in particular a polyol selected from glycerol, sorbitol, propylene glycol, xylitol, lactitol, or mixtures thereof. Sorbitol is especially useful as a humectant since it may also function as a sweetener.

Preferred humectants include one or more of propylene glycol and sorbitol. Preferably, a combination of propylene glycol and sorbitol are used.

When propylene glycol is used as a humectant it is preferably present in an amount of from about 0.5 to about 10% w/w; from about 1 to about 9% w/w; from about 2 to about 8% w/w; from about 3 to about 7% w/w; from about 4 to about 6% w/w; from about 4.5 to about 5.5% w/w; for example 5% w/w.

When sorbitol is used as a humectant it is preferably present in an amount of from about 1 to about 30% w/w; from about 5 to about 25% w/w; from about 10 to about 20% w/w; from about 11 to about 19% w/w; from about 12 to about 18% w/w; from about 13 to about 17% w/w; from about 14 to about 16% w/w; from about 14.5 to about 15.5% w/w; for example 15% w/w.

The toothpaste composition of the invention may include one or more abrasives, e.g. particulate abrasive material such as a silica, alumina, calcium carbonate, dicalcium phosphate, calcium pyrophosphate, hydroxyapatite, trimetaphosphate, insoluble hexametaphosphate, or mixtures thereof A preferred abrasive material is precipitated calcium carbonate.

The amount of abrasive material may be from about 1 to about 60% w/w; from about 5 to about 55% w/w; from about 10 to about 50% w/w; from about 15 to about 45% w/w; from about 20 to about 40% w/w; from about 25 to about 35% w/w; for example 30% w/w.

The toothpaste composition of the invention may include one or more thickening agents. Such thickening agents include, but shall not be limited to carboxylated polysaccharides. Carboxylated polysaccharides include a cellulose derivative, in particular a carboxyalkyl cellulose, and especially carboxymethylcellulose (CMC), typically used as its sodium salt (SCMC). The carboxylated polysaccharide serves to thicken the toothpaste, but may also contribute to the sensory properties of the toothpaste.

The amount of thickening agent may be from about 0.5 to about 2 % w/w; be from about 0.5 to about 1.9 % w/w; from about 0.6 to about 1.8 % w/w; from about 0.7 to about 1.7% w/w; from about 0.8 to about 1.6% w/w; from about 0.9 to about 1.5% w/w; from about 1 to about 1.4% w/w; from about 1.1 to about 1.3% w/w; for example 1.2% w/w.

The toothpaste of the present invention is advantageous in that, *inter alia,* it has antibacterial properties. In particular the toothpaste has antibacterial properties against gram positive bacteria. Notably, the toothpaste of the invention is effective against one or more of *Staphylococcus aureus, Bacillus cereus, Micrococcus luteus, Enterococcus faecalis, Staphylococcus epidermidis* and *Staphylococcus aureus* (MRSA) (see Table 1).

Furthermore, the propolis extract used in the toothpaste of the invention will generally comprise othe propolis extract used in the toothpaste of the invention will generally include one or more of the isoflavonoids isoliquiritigenin, liquiritigenin, formononetin, biochanin A, vestitol and pterocarpans (see Table 2).

In addition, the propolis extract used in the toothpaste of the invention will generally comprise prenylated benzophenones the propolis extract used in the toothpaste of the invention will generally include one or more of the prenylated benzophenones, selected from the group including nemorosone, propolona A and guttiferone (see Table 3).

Also, the propolis extract used in the toothpaste of the invention will generally comprise othe propolis extract used in the toothpaste of the invention will generally include one or more of the triterpenes selected from the group including one or more of β-amyrin, lupeol, *p*-amyrin, cycloartenol and lupeol acetate (see Table 4).

Although not essential, other ingredients that may be incorporated into the toothpaste composition of the invention include:

plaque buffers, such as urea, calcium lactate, calcium glycerophosphate and strontium polyacrylates;
desensitising agents, such as potassium citrate, potassium chloride, potassium tartrate, potassium bicarbonate, potassium oxalate, potassium nitrate and strontium salts;
anti-calculus agents, such as alkali-metal pyrophosphates, hypophosphite-containing polymers, organic phosphonates and phosphocitrates;
bio-molecules, such as bacteriocins, antibodies, and enzymes;
proteinaceous materials, such as collagen;
preservatives;
opacifying agents;
colouring agents;
pH-adjusting agents;
sweetening agents, such as saccharin and sorbitol (vide supra);

bleaching agents, such as peroxy compounds (e.g. potassium peroxydiphosphate);
buffers and salts to buffer the pH and ionic strength of the composition; and
pharmaceutically acceptable carriers, e.g. starch, sucrose, water or water/alcohol systems.

Water is a particularly preferred pharmaceutically acceptable carrier and is typically present in compositions of the invention at from 10 to 40%, in particular 15 to 35%, and especially at from 20 to 30% w/w of the total composition.

In a further aspect of the present invention, there is provided a toothpaste composition comprising from 0.5% w/w to 2% w/w of soft extract propolis, as hereinbefore described, a humectant, an abrasive and a thickening agent.

In a yet further aspect of the present invention, there is provided a method of cleaning teeth comprising brushing with a toothpaste composition comprising from 0.5% w/w to 2% w/w of soft extract propolis as hereinbefore described.

According to a yet further aspect of the present invention, there is provided a method of cleaning teeth comprising brushing with a toothpaste composition comprising from 0.5% w/w to 2% w/w of soft extract propolis, as hereinbefore described, a humectant, an abrasive and a thickening agent.

The present invention also provide a method of treatment or prevention of one or more of dental caries, periodontopathies and candidiasis which comprise the use a toothpaste composition as hereinbefore described.

The present invention also provides a method of preparing a toothpaste composition as hereinbefore described which comprises mixing from 0.5% w/w to 2% w/w of a soft extract propolis with a humectant, an abrasive and a thickening agent. The invention further provides a method of preparing a soft extract of propolis for use in a toothpaste composition or a method as hereinbefore described which comprises the extraction of raw propolis with an alcohol.

The soft extract of propolis may be prepared by the extraction of raw propolis with alcohol, such as ethanol. Preferably, the raw propolis is mascerated, comminuted or otherwise deagglomerised into particles which generally have a diameter of about 1cm or less. It will be understood by the person skilled in the art that the treated raw propolis will have a particle size distribution such that ≥75% w/w of the propolis particles have diameter of about 1cm or less. It will be understood by the person skilled in the art that the particle size distribution of the comminuted raw propolis may vary and may be such that ≥76% w/w of the propolis particles have diameter of about 1cm or less; or ≥77% w/w of the propolis particles have diameter of about 1cm or less; or ≥78% w/w of the propolis particles have diameter of about 1cm or less; or ≥79% w/w of the propolis particles have diameter of about 1cm or less; or ≥80% w/w of the propolis particles have diameter of about 1cm or less; or ≥85% w/w of the propolis particles have diameter of about 1cm or less; or ≥90% w/w of the propolis particles have diameter of about 1cm or less; or ≥95% w/w of the propolis particles have diameter of about 1cm or less.

Thus according to this aspect of the invention there is provided a method of preparing a soft extract of propolis which comprises comminuting raw propolis into particles such that ≥75% w/w of the propolis particles have a diameter of about 1cm or less.

If necessary, the raw propolis may be frozen, for example to about -20°C, in order to aid the comminution of the raw propolis into the particles as hereinbefore described.

The procedure for the manufacture of a soft extract of propolis is described in more detail.

A soft extract of propolis is the end product obtained by selective extraction of the active substances from propolis raw material. A 70% v/v ethanol/ water mixture is preferably used as solvent, to obtain a viscous product comprising from about 85 to about 90% w/w of propolis in ethanol/ water. A quality-graded soft extract should be perfectly soluble in 70% ethanol/ water, without solid waste materials. The yield of the process may vary, depending upon, *inter alia,* the propolis type or source, according its content of wax and residuals. A mean yield of 100g of soft extract per kg of raw propolis is estimated.

The soft extract of propolis may be purified using the Pichansky method. Thus, for example, the gross (or raw) propolis may be macerated in an ethanol/ water solution. Gross (or raw) propolis VS alcohol based on % of weight/volume is used. The final quality of the product is observed through the presence of total solids. The first extraction is gross propolis 50 kg and alcohol at 70% v/v (ethanol/ water mixture), filtered at a low concentration csp 100 litres. In subsequent extractions, residual propolis is used with 70% v/v ethanol.

Soft propolis extract is obtained by selective extraction of the active substances of propolis raw material by 70° proof ethanol (i.e. 40% ethanol/water mixture), used as solvent, to obtain a viscous product with a concentration between 85 and 90 % w/w of propolis, the remainder being made ethanol and water.

A soft quality summary must be perfectly soluble in 70° proof ethanol, without generating solid residues. The performance of the propolis to another, depending on its content of wax and other residues. It is estimated that an average of about 100 g of soft propolis extract yield per kg of raw, unpurified propolis.

The invention will now be described by way of example only. The described method that follows applies to an amount of 5 to 100 kg of raw material:

**Example 1**

**Preparation of soft propolis extract**

The procedure for the manufacture of a soft extract of propolis is described in more detail.
The following method is applied to an amount of 5-100kg of raw material.

1. **Raw propolis:**

Raw propolis (of organic quality) was collected by beekeepers through a method of scraping. Desirably the propolis does not have detectable traces of wax or mechanical impurities and does not show any signs of degradation.

If necessary, the raw propolis can be stored at from 5 to 10°C, protected from light and moisture, for example, in plastic bottles (latex should be avoided).

2. **Grinding or Fragmentation/ Extraction**

**RP:** weight of raw propolis to be processed
Propolis pieces will be reduced to particles of about 1cm in diameter or less, by means of grinding. If necessary, the propolis can be frozen, prior to grinding, to about -20°C.
A crusher employed will generally be a double walled bowl. A warm fluid, e.g. water, is circulated between the walls, for example, at a temperature of about 45°C. A system of rotating/ mixing is provided inside the crusher.
The crushed or fragmented raw propolis (weight of RP) is mixed with ethanol in a proportion of about 1litre of ethanol per 600g of raw propolis. The mixture is shaken or stirred at about 50 rpm and then cooled to about 15°C.

3. **Filtering**

The mixture is then filtered using a tissue filter under vacuum. It is important that the filtering process is conducted at room temperature (e.g. about 20°C) with a cooled solution, ensuring that all wax is precipitated by solidification. If the mixture is too warm, this can give rise to a risk of wax being transferred to the solution of the raw propolis extract.
The remaining ethanol is allowed to evaporate from the filter and the residuals are weighed (R is the weight of residuals obtained after alcohol evaporation).

4. **Concentration**

To obtain the Propolis Soft Extract, the filtered extract is concentrated by means of vacuum evaporation, recovering most of the alcohol from the solution. The extract is concentrated in a vacuum evaporator at a temperature below 50°C, ensuring the quality of the end product.
To finish the process, the soft extract is weighted. PSE is the weight of the soft extract.
The content of active material C% of the produced soft extract will be:

$$C\% = (RP - R)/PSE$$

This value should be from about 85% to about 90% w/w.

$$C\%min = 85\% \leq C\% \leq 90\% = C\%max.$$

Propolis soft extract should be pasty and sticky, like a glue.
If the alcohol content is too high, the extract may be runny or too liquid. If the alcohol content is too low then the extract may tend to solidify and become hard and unsuitable for use in a toothpaste formulation.
It is required that alcohol evaporation runs until the PSE reaches a level of C% from 85 to 90%, that means, its weight is between:

$$(RP-R)/0,9 \leq PSE \leq (RP-R)/0,85$$

Concentration factor of raw propolis related to PSE will be:

$$CF = RP / PSE$$

**Example 2**

**Propolis Toothpaste Formulation**

Each 100g contains:

| Ingredient | Amount (g) |
|---|---|
|  |  |
| Soft extract of propolis (Dun) | 1 |
| Propylene glycol | 5 |
| Sorbitol 70% | 15 |
| Silicate solution of Na 41 Be | 2 |
| Sodium saccharin | 15 |
| Flavour* | 0.7 |
| Sodium carboxymethyl cellulose | 1.2 |
| Precipitated calcium carbonate light | 30 |
| Solution of sodium lauryl sulphate at 33% | 5.6 |
| Light mineral oil | 1 |
| Purified water csp | 23.5 |
|  |  |
| Total | 100g |
| *Eucalyptus, cinnamon, strawberry, pineapple, raspberry and/or anise flavours may be added. | |

**Table 1**

| Sample No. | *Staphylococcus aureus* | *Bacillus cereus* | *Micrococcus luteus* | *Enterococcus faecalis* | *Staphylococcus epidermidis* | *Staphylococcus aureus* (MRSA) | *Klebsiella sp* | *E.coli* |
|---|---|---|---|---|---|---|---|---|
| | | | | | | | | |
| 33 | 24.5 | 20.0 | 30.0 | 20.0 | 25.0 | 29.5 | NI | NI |
| 6 | 25.0 | 22.5 | 20.0 | 20.0 | 30.0 | 33.3 | NI | NI |
| 61 | 26.5 | 20.0 | 25.0 | 20.0 | 20.0 | 25.0 | NI | NI |
| 15 | 25.0 | 22.5 | 25.0 | 20.0 | 21.0 | 25.0 | NI | NI |
| 9 | 17.5 | 22.5 | 30.0 | 20.0 | 20.0 | 17.5 | NI | NI |
| 29 | 19.5 | 16.5 | 30.0 | 20.0 | 20.0 | 17.5 | NI | NI |
| 35 | 19.5 | 20.0 | 35.0 | 20.0 | 17.5 | 23.0 | NI | NI |
| 42 | NI | 10.0 | 10.0 | 12.0 | NI | NI | NI | NI |
| 8 | 10.0 | 9.0 | NI | 15.0 | NI | 10.0 | NI | NI |
| 21 | 5.0 | 5.0 | NI | 10.0 | NI | NI | NI | NI |
| 38 | 5.0 | 10.0 | NI | 12.0 | NI | NI | NI | NI |
| | | | | | | | | |

**Table 2**

| Isoflavonoids pharmacological activities | |
|---|---|
| **Propolis Type** | **Pharmacological Activity** |
| | |
| Isoliquiritigenin | antineoplastic |
| | Antinflamatory |
| | antifungal |
| Liq uiritigenin | Antinflamatory |
| Formononetin | Estrogenic activity |
| Biochanin A | Estrogenic activity |
| Vestitol | Antibacterial *(Helicobacter pylori)* |
| Pterocarpans | Antifungal |
| | Antibacterial |
| | Transcriptase inhibitor |
| | |

**Table 3**

| Pharmacological activities of prenylated benzophenones | |
|---|---|
| **Prenylated Benzophenones** | **Pharmacological Activity** |
| | |
| nemorosone | Antitumora |
| | Antioxidant |
| | antiviral |
| Propolona A | Antimicrobial |
| guttiferone | reverse transcriptase Inhibitors |
| | |

**Table 4**

| pharmacological activities of triterpenes | |
|---|---|
| **Triterpene** | **Pharmacological Activity** |
| | |
| β-amyrin | antiinflammatory |
| | analgesic |
| | antifungal |
| | Anxiolytic and antidepressant |
| | hepatoprotector |
| | antimicrobial |
| | antiulcer |

(continued)

| pharmacological activities of triterpenes | |
|---|---|
| **Triterpene** | **Pharmacological Activity** |
| lupeol | antineoplastic |
| | antiinflammatory |
| | hypercholesterolemic |
| | antifungal |
| | analgesic |
| p-amyrin | antiinflammatory |
| cycloartenol | antiinflammatory |
| Lupeol acetate | antiulcer |

**Claims**

1. A toothpaste composition comprising from 0.5% w/w to 2% w/w of soft extract of propolis wherein the soft propolis extract comprises from 85 to 95% w/w of active material.

2. A toothpaste composition according to claim 1 wherein the content of active material (C%) of the soft propolis extract is from about 85% to about 90% w/w.

3. A toothpaste composition according to any one of claims 1 or 2 wherein the composition includes one or more humectants.

4. A toothpaste composition according to any one of the preceding claims wherein the composition includes one or more abrasives.

5. A toothpaste composition according to any one of the preceding claims wherein the composition includes one or more thickening agents.

6. A toothpaste composition according to any one of the preceding claims which has antibacterial properties against gram positive bacteria.

7. A toothpaste composition according to any one of the preceding claims wherein the propolis extract comprises one or more isoflavonoids.

8. A toothpaste composition according to any one of the preceding claims wherein the propolis extract comprises orenylated benzophenones.

9. A toothpaste composition according to any one of the preceding claims wherein the composition comprises one or more triterpenes.

10. A toothpaste composition according to claim 1 comprising from 0.5% w/w to 2% w/w of soft extract propolis, a humectant, an abrasive and a thickening agent.

11. A toothpaste composition according to any one of the preceding claims wherein each 100g of the composition contains:

| Ingredient | Amount (g) |
|---|---|
| | |

(continued)

| Ingredient | Amount (g) |
|---|---|
| Soft extract of propolis (Dun) | 1 |
| Propylene glycol | 5 |
| Sorbitol 70% | 15 |
| Silicate solution of Na 41 Be | 2 |
| Sodium saccharin | 15 |
| Flavour* | 0.7 |
| Sodium carboxymethyl cellulose | 1.2 |
| Precipitated calcium carbonate light | 30 |
| Solution of sodium lauryl sulphate at 33% | 5.6 |
| Light mineral oil | 1 |
| Purified water csp | 23.5 |
| | |
| Total | 100g |

12. A method of cleaning teeth comprising brushing with a toothpaste composition according to any one of the preceding claims.

13. A method of treatment or prevention of one or more of dental caries, periodontopathies and candidiasis which comprises the use a toothpaste composition according to any one of claims 1 to 11.

14. A method of preparing a soft extract of propolis for use in a toothpaste composition which comprises the extraction of raw propolis with an alcohol.

15. A method of preparing a soft extract of propolis according to claim 14 which comprises mascerating or comminuting raw propolis raw propolis into particles which generally have a diameter of about 1cm or less.

Europäisches
Patentamt

European
Patent Office

Office européen
des brevets

**EUROPEAN SEARCH REPORT**

Application Number

EP 13 00 2760

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/029015 A2 (UNIV DE LA FRONTERA [CL]; PONTIFICIA UNIVERSIDAD CATOLICA DE CHILE [CL] 8 March 2012 (2012-03-08) * claims 1-4 * * example 1 * ----- | 1-15 | INV. A61K8/98 A61Q11/00 |
| X | US 2012/288454 A1 (ZHAO WENZHONG [CN]) 15 November 2012 (2012-11-15) * paragraph [0052] * ----- | 1-15 | |
| X | FR 2 932 386 A1 (DOHAN DAVID MARCEL [FR]; VERVELLE ALAIN GERMAIN [FR]) 18 December 2009 (2009-12-18) * examples 1,3 * * claims 1,2 * ----- | 1-13 | |
| X | WO 2006/109344 A2 (GASPARINI GIANLUIGI [IT]; MONTI ANDREA [IT]) 19 October 2006 (2006-10-19) * page 3, line 16 - line 20 * * page 4, line 20 - line 31 * * claims 1-6 * ----- | 1-13 | |
| | | | TECHNICAL FIELDS SEARCHED (IPC) A61K A61Q |

The present search report has been drawn up for all claims

| Place of search | Date of completion of the search | Examiner |
|---|---|---|
| Munich | 5 November 2013 | Cismaru, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 00 2760

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2013

| Patent document cited in search report | | Publication date | Patent family member(s) | | Publication date |
|---|---|---|---|---|---|
| WO 2012029015 | A2 | 08-03-2012 | EP<br>US<br>WO | 2612667 A2<br>2013280183 A1<br>2012029015 A2 | 10-07-2013<br>24-10-2013<br>08-03-2012 |
| US 2012288454 | A1 | 15-11-2012 | CN<br>US<br>WO | 102858308 A<br>2012288454 A1<br>2012068730 A1 | 02-01-2013<br>15-11-2012<br>31-05-2012 |
| FR 2932386 | A1 | 18-12-2009 | NONE | | |
| WO 2006109344 | A2 | 19-10-2006 | NONE | | |

EPO FORM P0459

For more details about this annex : see Official Journal of the European Patent Office, No. 12/82